# EUROPEAN PATENT SPECIFICATION

(11) **EP 0 461 285 B1**
(45) Date of publication and mention of the grant of the patent: **13.09.1995**
(21) Application number: 90111197.1
(22) Date of filing: 13.06.1990
(51) Int. Cl.: C07D 303/04, C07D 303/14, C07C 47/21, A01N 43/04

(54) **Hyphantria cunea sex pheromones, intermediates for their synthesis and attractant compositions including these pheromones**
Hyphantrea cunea Sexpheromone, Zwischenprodukte ihrer Synthese und Lockstoffzusammensetzungen auf der Basis dieser Pheromone
Hyphantria cunea phéromones sexuelles, des produits intermédiaires de leurs synthèse ainsi que des compositions attiractives basées sur ces phéromones

(43) Date of publication of application: 18.12.1991
(73) Proprietor: Nitto Denko Co. Ltd., Ibaraki City, Osaka 567 (JP)
(72) Inventor: Toth, Miklos, Dr., H-1029 Budapest 11 (HU); Buser, Hans-Rudolf, Dr., CH-8820 Wädenswil (CH); Arn, Heinrich, Dr., CH-8832 Wollerau (CH); Mori, Kenji, Bunkyoku, Tokyo 113 (JP); Ninomiya, Yasuo, Nitto Denko Co., Ltd., Ibaraki City, Osaka 567 (JP); Omata, Tetsuo, Nitto Denko Co., Ltd., Ibaraki City, Osaka 567 (JP); Senda, Shuji, Nitto Denko Co., Ltd., Ibaraki City, Osaka 567 (JP); Takeuchi, Tadashi, Himi City, Toyama, 935 (JP); Aburatani, Masakazu, Takaoka City, Toyama, 933 (JP)
(74) Representative: Hansen, Bernd, Dr. Dipl.-Chem.

(56) References cited:
- Liebigs Annalen der Chemie vol. 5, 1989, Weinheim pages 453 - 457; Kenji Moriet al.: "Synthesis of the Enantiomers of 3Z,6Z -cis-9,10-Epoxy-1,3,6-henicosatriene and 3Z,6Z -cis-9,10-Epoxy-1,3,6-icosatriene,the New PheromoneComponents of Hyphantria cunea"
- Tetrahedron Letters vol. 30, no. 26, 1989, Great Britain pages 3405 - 3408; M.
- Toth et al.: "Identification of 3Z,6Z -1,3,6-9,10-Epoxyheneicosatriene and3Z,6Z -1,3,6-9,10-Epoxyeicosatriene in the sex Pheromone of Hyphantria cunea"
- Tetrahedron vol. 42, no. 13, 1986, Great Britain pages 3471 - 3478; Kenji Moriet al.: "Syntheses of optically active Pheromones with an Epoxy ring, + -Disparlure and both the Enantiomers of 3Z,6Z -cis-9,10-Epoxy-3,6-heneicosadiene"
- Comptes Rendus Hebdomadaires des Séances de l'Académie des Sciences vol. 294,no. II, 04 January 1982, Paris pages 41 - 44; Jacques Einhorn et al.:"Isolement et identification de la phéromone sexuelle attractive de Hyphantriacunea Drury Lépidoptère,Arctiidae ."
- Journal of Chemical Ecology vol. 8, no. 2, February 1982, pages 383 - 396; A.S.Hill et al.: "Sex Pheromone of the Fall Webworm Moth,Hyphantria cunea"

## Description

The present invention relates to compositions including Hyphantria cunea sex pheromone.

The object of the invention is to provide attractant components having a high attraction effect for Hyphantria cunea.

Hyphantria cunea, whose original home is North America, causes various damage to trees, farms and fruit by its parasitic activity in many countries of the world such as the United States, the Soviet Union, Europe etc.

Larvae of Hyphantria cunea were first found in Japan just after World War II, in Tokyo.

After this finding the Japanese Government tried to eradicate them from Japan at an early stage.

However, it proved impossible to prevent an increase in their distribution and consequently they are now to be found not only in the Kanto area but also in the Kansai area and the Tohhoku area.

To date, they are widely distributed in Japan, centring mainly in urban areas where they cause extensive damage to trees.

The use of sex pheromones for protecting and removing Hyphantria cunea was investigated and scholars from the United States and the Soviet Union were involved in cooperative research analyzing the structures of the sex pheromones.

W.L. Roelofs et al reported that (9Z,12Z)-9,12-octadecadien-1- al (formula 4), (9Z,12Z,15Z)-9,12,15-octadecatrien-1- al (formula 5) and (3Z,6Z)-cis-9,10-epoxy-3,6-heneicosadiene (formula 6) were sex pheromones of Hyphantria cunea (A.S. Hill, B.C. Kovalev, L.N. Nikolaeva, W.L. Roelofs, J.Chem. Ecol.8, 383 (1982)).

In attractant tests for Hyphantria cunea carried out in Japan and Europe using the above-mentioned 3 components the attraction effect was found to be very weak so that it was impossible to make practical use of the attractant compositions as traps to catch Hyphantria cunea.

M. Toth et al (Tetrahedron Letters, Vol. 30, No. 26, pp. 3405-3408) reported that (3Z,6Z)-1,3,6-9,10-epoxyheneicosatriene (formula 1) and (3Z,6Z)-1,3,6-9,10-epoxyeicosatriene (formula 2) were identified in the sex pheromone gland of H. cunea. They found that the mixture comprising only the compounds having the formulas (1) and (2) or the above-mentioned ternary blend (formulas (4), (5) and (6)) were not attractive.

C.R. Acad.Sc. Paris, t 294 (04.01.1982) discloses a composition comprising the compounds having the formulas (4) and (5) and additionally (Z,Z)-3,6-(cis)-9,10-epoxyheneicosadiene (formula (6)). However, as disclosed in the former article, such a composition is not attractive.

The present inventors conducted an earnest study to create attractant components having a high attractive effect for H. cunea, i.e. H. cunea sex pheromones having high attraction activity and, furthermore, attractant compositions including the above-mentioned pheromones with a high attractive effect for H. cunea.

### BRIEF DESCRIPTION OF THE DRAWINGS

Fig. 1 is the gas chromatogram of the extraction of ovipositor of H. cunea females using a flame ionization (FID) and an electro antenographic detector (EAD).

Fig. 2 charts the results of mass spectric analysis of (3Z,6Z)-cis-9,10-epoxy-1,3,6-eicosatriene (formula 2). 9,10-epoxy-1,3,6-eicosatriene (formula 2).

### DETAILED DESCRIPTION OF THE INVENTION

*Hyphantria cunea* sex pheromones relating to this invention are (3Z,6Z)-cis-9,10-epoxy-1,3,6-heneicosatriene (formula 1) and (3Z,6Z)- cis-9,10-epoxy-1,3,6-eicosatriene (formula 2). 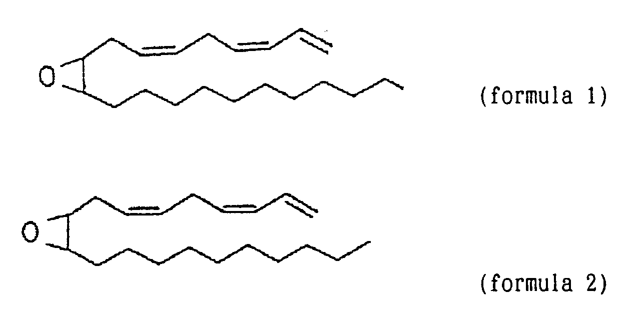
The physical properties of (3Z,6Z)-cis-9,10-epoxy-1,3,6-heneicosatriene (formula 1) are as follows: the melting point is 14∼15°C, the refractive index with sodium D light ray at 16°C is 1.4781, and the specific rotation, [α] ¹⁶_{D} with sodium D light ray of (3Z,6Z,9S,10R)-cis-9,10- epoxy-1,3,6-heneicosatriene (formula 1a) as the (9S,10R) enantiomer of heneicosatriene is -0.41° (c=1.97, in CHCl₃ ). 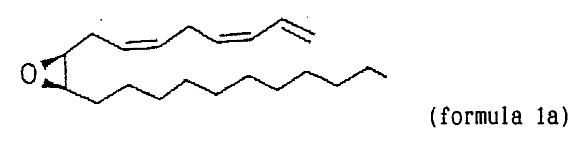
The physical properties of (3Z,6Z)-cis-9,10-epoxy-1,3,6-eicosatriene (formula 2) are as follows:the melting point is 2∼3 °C, the refractive index with sodium D light ray at 16°C is 1.4787, and the specific rotation, [α] ¹⁶_{D} with sodium D light ray at 16 °C of (3Z,6Z,9S,10R)-cis-9,10-epoxy-1,3,6-eicosatriene (formula 2a) as the (9S,10R) enantiomer of eicosatriene is - 0.41° (c=1.23, in CHCl₃ ). 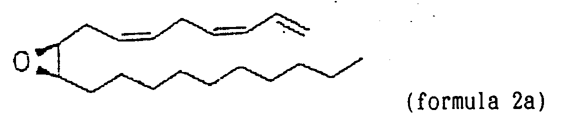
In the first place, the present inventors have confirmed that (3Z,6Z)-cis-9,10-epoxy-1,3,6-heneicosatriene (formula 1) and (3Z,6Z)-cis-9,10-epoxy-1,3,6-eicosatriene (formula 2) were real *Hyphantria cunea* sex pheromones by the following procedures.

Larvae of *Hyphantria cunea* were gathered and reared from larvae to pupae.

The ovipositor tips of one hundred adult *Hyphantria cunea* females being selected from the 2 days old metamorphosed females were taken out, and their pheromones extracted with hexane (1∼5 µl per 1 female).

Continuously, the extraction obtained by above the process was identified according to the following manner.

First, the gas chromatograph analysis was operated on a SE54 column with a flame ionization detector (FID) and an electro antennographic detector (EAD).

The operation of gas chromatograph analysis was carried out with temperature conditions being maintained at 40°C for 2 minutes then raised to 140°C by 20°C/min and further raised to 225°C by 5°C/min

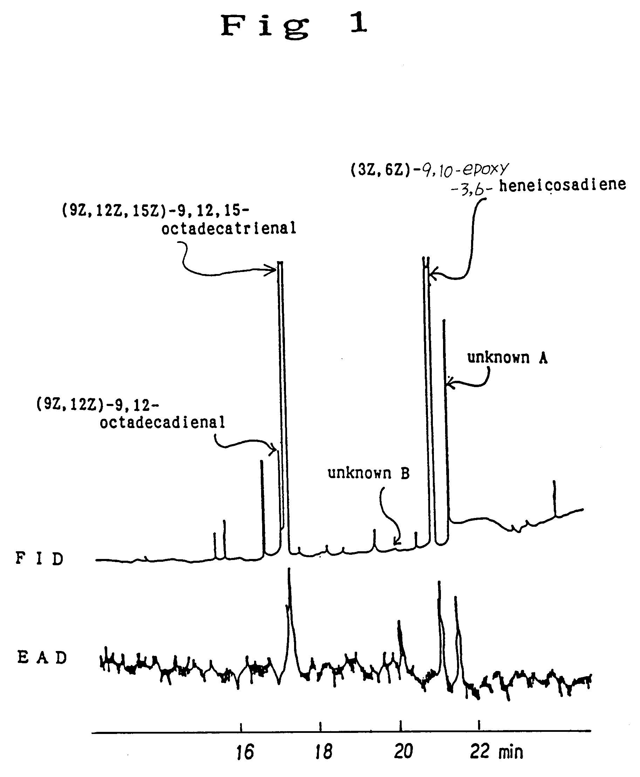

The results are shown in Fig. 1.

Using the gas chromatograph with the electro antennographic detector (EAD), the presence of 5 biologically active components are analyzed.

Furthermore, each fraction separated by gas chromatography was subjected to mass spectrometry, i.e., GC-MS analysis.

The operation of GC-MS analysis (Finnigan 4023, EI, 50eV) was carried out on a SE54 with a program of maintaining temperature conditions at 50 °C for 2 minutes, then raising up to 140 °C by 20°C/min and further raising up to 280 °C by 5 °C/min.

Subsequently, 3 components of 5 biologically active components were known sex pheromone of *Hyphantria cunea* which were (9Z, 12Z)-9,12-octadecadien-1-al (formula 4) , (9Z,12Z,15z)- 9,12,15-octadecatrien -1-al (formula 5) and (3Z,6Z)-cis-9,10-epoxy-3,6-heneicosadiene (formula 6). 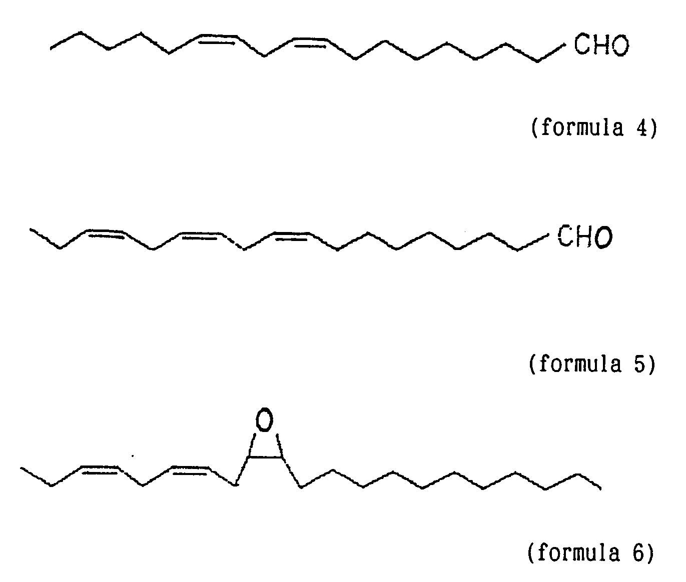
According to the present invention, the attractant composition includes the compounds having the formulas (1), (4) and (5) wherein (3Z,6Z)-3,6-9,10-epoxyeicosadiene, (3Z,6Z,9Z)-3,6,9-heneicosatriene, and (3Z,6Z,9Z)-1,3,6,9-heneicosatetraene are excluded. Optionally, the compounds having the formulas (2) and/or (6) may also be contained therein.

Furthermore, the identification of the two unknown biologically active components A and B (see Fig. 1) was conducted as follows.

The unknown component A was subjected to gas chromatograph analysis and measured 2289 in RI by the electro antennographic detector (EAD).

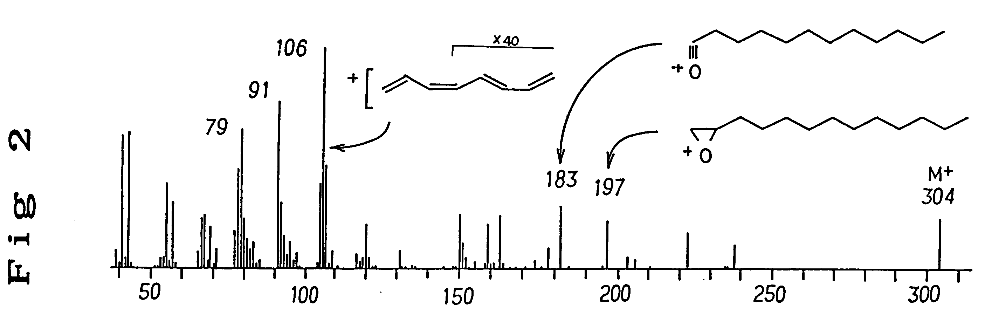

The mass spectrum of the unknown component A showed a molecular ion peak at m/e 304 with a series of highly unsaturated ion peaks at m/e 79, 91 and 106 (see fig. 2).

On the other hand, the mass spectrum of (3Z,6Z)-cis-9,10-epoxy-3,6-heneicosadiene (formula 6) showed ion peaks at m/e 79, 93 and 108, suggesting a terminal double bond of the unknown component A.

Also ion peaks at m/e 183 and 197 suggested the presence of epoxy groups in 9 and 10 positions.

Moreover, the retention difference between the unknown component A and (3Z,6Z)-9,10-epoxy-3,6-heneicosadiene (formula 5) was the same as between (3Z,6Z,9Z)-1,3,6,9-nonadecatetraene and (3Z,6Z,9Z)-3,6,9-nonadecatriene, suggesting double bond conjugation of the unknown component A.

In order to test the above hypothesis, (3Z,6Z,9Z)-1,3,6,9-nonadecatetraene was epoxidized with m-chloroperbenzoic acid.

This reaction mixture was subjected to gas chromatograph analysis with the electro antennographic detector (EAD) and results showed that the mixture contained three components.

One of the above components proved highly active to the male *Hyphantria cunea*.

Furthermore, the mass spectrum of this component gave a molecular ion peak at m/e 276 and ion peaks at m/e 79, 91 and 106, thus suggesting that the unknown component A is the C₂₁ homologue .

The physical properties of the unknown component A are identical with those of (3Z,6Z)-cis-9,10-epoxy-1,3,6-heneicosatriene (formula 1).

Thus, the unknown component A is confirmed to be (3Z,6Z)-cis-9,10-epoxy-1,3,6-heneicosatriene (formula 1).

(3Z,6Z)-cis-9,10-epoxy-1,3,6-heneicosatriene (formula 1) obtained by separation and purification had a yield of 8 ng per 1 female *Hyphantria cunea* . 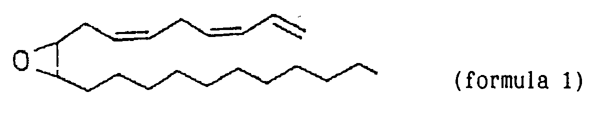
The unknown component B is identified in the same manner as above.

The unknown component B is subjected to gas chromatograph analysis and measured 2185 in RI by the electro antenographic detector (EAD).

Furthermore, the mass spectrum of the unknown component B shows a molecular ion peak m/e 260 and a highly unsaturated compound ion peaks at m/e 79, 91 and 106.

Furthermore, the physical properties of the unknown B are identical with those of (3Z,6Z)-cis-9,10-epoxy-1,3,6-eicosatriene (formula 2).

Moreover, a component comprising (9S,10R) eicosatriene isolated from the unknown component B measured for specific rotation with sodium D light ray at 16°C, which is identical with that of (3Z,6Z)-cis-9,10-epoxy-1,3,6-eicosatriene (formula 2).

Thus, the unknown component B is confirmed to be the C₂₀ homologue, which is (3Z,6Z)-cis-9,10-epoxy-1,3,6-eicosatriene (formula 2).

(3Z,6Z)-cis-9,10-epoxy-1,3,6-eicosatriene (formula 2) obtained by separation and purification had a yield of 0.04 ng per 1 female *Hyphantria cunea*.

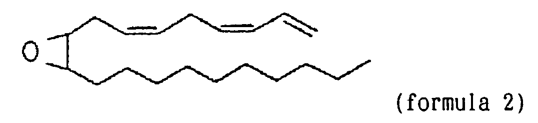

The activities are measured by means of the electro antennographic detector (EAD) of (3Z,6Z)-cis-9,10-epoxy-1,3,6-heneicosatriene (formula 1) and (3Z,6Z)-cis-9,10-epoxy-1,3,6 -eicosatriene (formula 2) comprising the (9S, 10R) enantiomer, i.e., (3Z,6Z,9S,10R)-cis-9,10-epoxy-1,3,6-heneicosatriene (formula 1a) and (3Z,6Z,9S,10R)-cis-9,10-epoxy-1,3,6-eicosatriene (formula 2a), respectively.

The results show that (3Z,6Z)-cis-9,10-epoxy-1,3,6-heneicsatriene (formula 1) and (3Z,6Z)-cis-9,10-epoxy-1,3,6-eicosatriene (formula 2) both with (9S,10R) heneicosatriene and eicosatriene are more active to the antenna of male *Hyphantria cunea* than with (9 R,10S) heneicosatriene and eicosatriene.

Accordingly, it is indicated that each component consisting of the S form in 9-position and the R-form in 10-position exhibits higher biological activities. 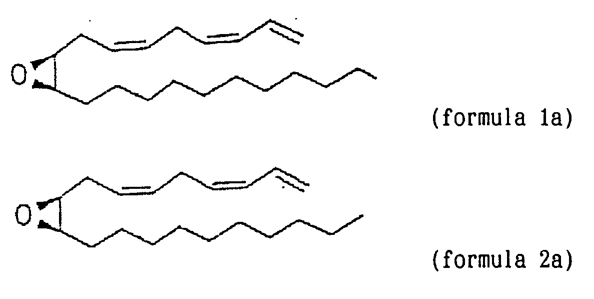
The above results reveal that both components of the present invention, (3Z,6Z)-cis-9,10-epoxy-1,3,6-heneicosatriene (formula 1) and (3Z,6Z)-cis-9,10-epoxy-1,3,6-eicosa-triene (formula 2), are biologically active pheromones, and that (3Z,6Z,9S,10R)-cis-9,10 -epoxy-1,3,6-heneicosatriene (formula 1a) and (3Z,6Z,9S,10R)-cis-9,10- epoxy-1,3,6-eicosatriene (formula 2a) are pheromones especially high in biological activities.

The inventive (3Z,6Z)-cis-9,10-epoxy-1,3,6-heneicosatriene (formula 1) and (3Z,6Z)-cis-9,10-epoxy-1,3,6-eicosatriene (formula 2) are much easier to synthesize from (5Z,8Z)-cis-2,3-epoxy-5,8,10-undecatrien-1-ol(formula 3). 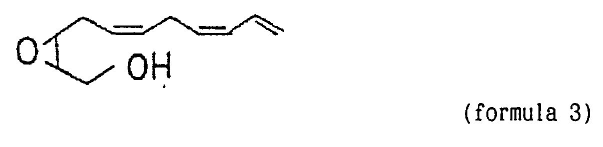
Stated more specifically, (5Z,8Z)-cis-2,3-epoxy-5,8,10-undecatrien-1-ol(formula 3) is allowed to react with tosylchloride (p-toluenesulfonyl chloride) in a pyridine solvent to prepare (3Z,6Z)-cis-9,10-epoxy-1,3,6-undecatrienyl tosylate (formula 7),
which is thereafter purified and reacted with lithium di(n-decyl)cuprate in an ether solvent at low temperature.

On completion of the reaction, the after-treatment was effected in accordance with the usual manner and the resultant was purified using a silica gel chromatography to give (3Z,6Z)-cis-9,10-epoxy-1,3,6-heneicosatriene (formula 1).

These steps are shown in following . 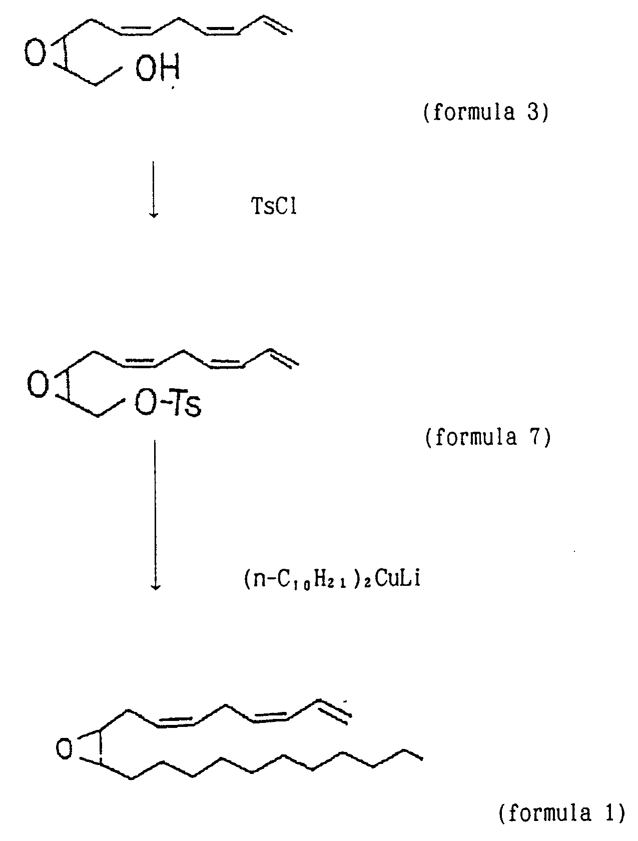
(5Z,8Z)-cis-2,3-epoxy-5,8,10-undecatrien-1-ol (formula 3) is treated to prepare (3Z,6Z)-cis-9,10-epoxy-1,3,6-undecatrienyl tosylate (formula 7) in the same manner as the above, which is thereafter reacted with lithium di(n-decyl) cuprate to prepare (3Z,6Z)-cis-9,10-epoxy-1,3,6-eicosatriene (formula 2), then after-treatment and purification are needed in the same manner as the above.

These steps are shown in following . 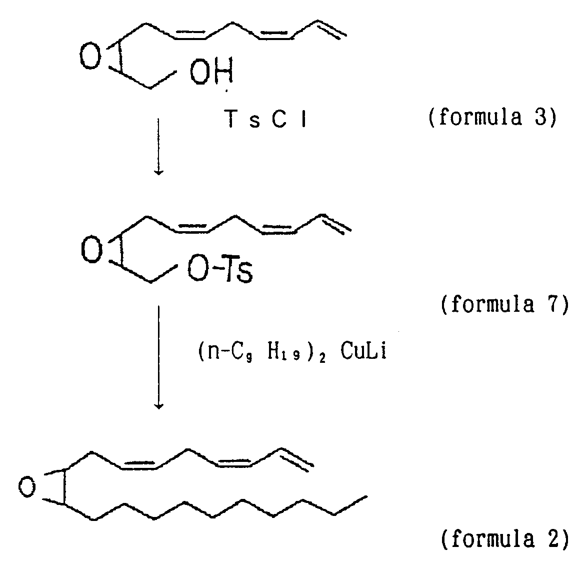
In these steps, (3Z,6Z,9S,10R)-cis-9,10-epoxy-1,3,6-heneicosa triene (formula 1a) or (3Z,6Z,9S,10R)-cis-9,10-epoxy-1,3,6-eicosatrien (formula 2a) is obtained by using (5Z,8Z)- cis-2,3-epoxy -5,8,10-undecatrien-1-ol (formula 3) being the (2R,3S) triene, i.e., (2R,3S,5Z,8Z)-cis-2,3-epoxy-5,8,10-undecatrien-1-ol (formula 3a). 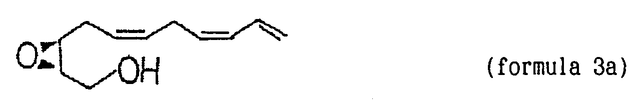
Further more, (3Z,6Z,9R,10S)-cis-9,10-epoxy-1,3,6-heneicosatriene (formula 1b) or (3Z,6Z,9R,10S)-cis-9,10-epoxy-1,3,6-eicosatrien (formula 2b) is obtained by using (5Z,8Z)-cis-2,3-epoxy-5,8,10 -undecatrien-1-ol (formula 3) being the (2S,3R) triene, i.e., (2S,3R,5Z,8Z)-cis-2,3-epoxy-5,8,10-undecatrien-1-ol (formula 3b).

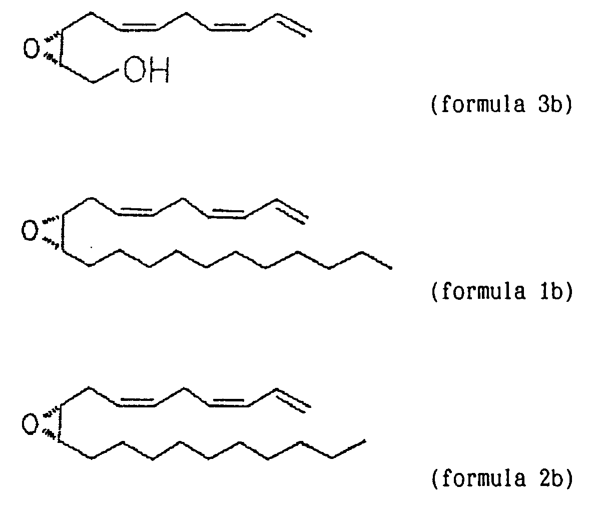

This inventional component of (5Z,8Z)-cis-2,3-epoxy-5,8,10-undecatrien-1-ol (formula 3) can be obtained by following the step 1 to 10. 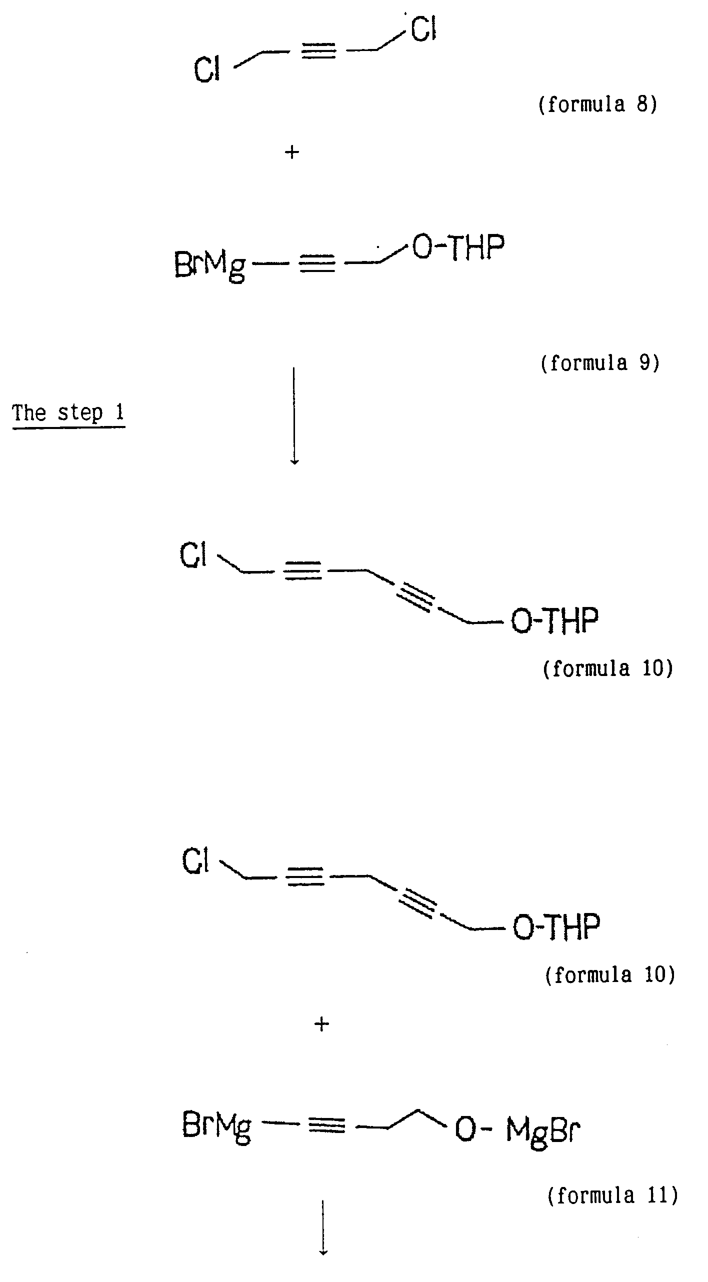 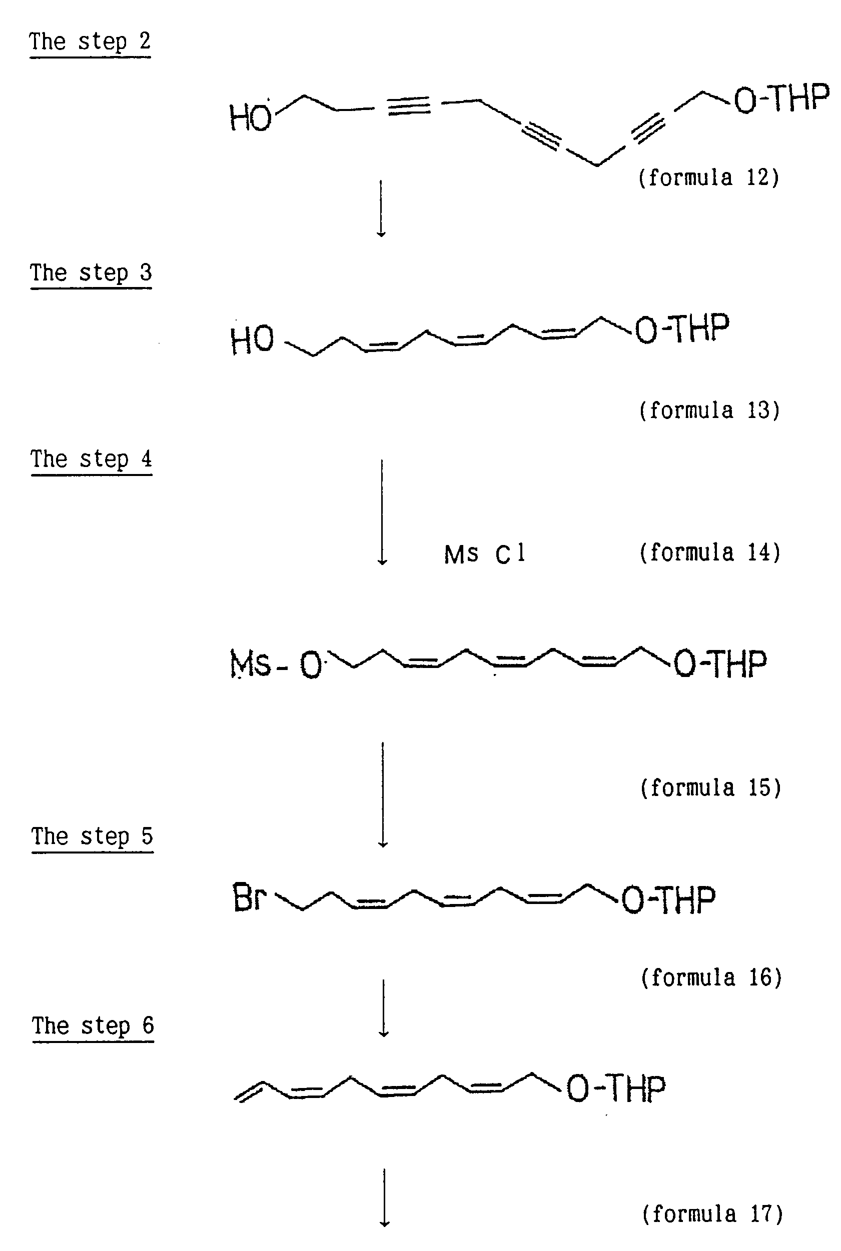 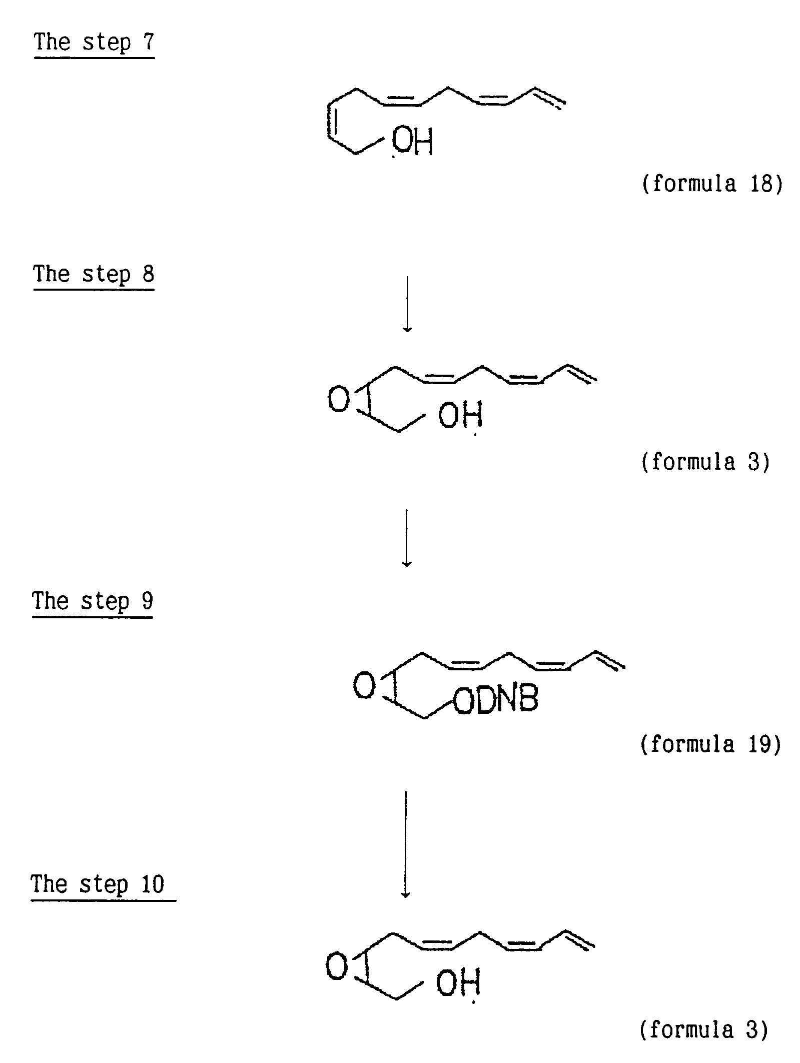
Hereinafter, the detailed explanation from the step 1 to 10 is described.

### [The step 1]

In summary of this step, the diyne compound (formula 10) is prepared from 1,4-dichloro-2-butyne (formula 8) which is allowed to react with the anion of propargyl alcohol derivative (formula 9)in the presence of copper salt.

The amount of the anion of propargyl alcohol derivative to be used is desirably 1∼1.1 equivalent weight to the amount of 1,4-dichloro-2-butyne (formula 8).

The monovalent copper salt is used in the necessary amount for a catalyst.

The amount of copper (I) chloride to be used is favourably 0.01∼ 0.2 equivalent weight to the amount of 1,4-dichloro-2-butyne.

It is preferable that this process is carried out with the ether system solvent especially tetrahydrofuran.

The desirable reaction temperature is 0 ∼50°C in the case of 1,4-dichloro-2-butyne (formula 8) which is allowed to react with the anion of propargyl alcohol derivative.

In this step, 1,7-bis-(2-tetrahydropyranyloxy)-2,5,8,-decatriene produced as by-products is to be separated by the silica gel chromatography.

### [The step 2]

In summary of this step, the toriyne compound (formula 12) is prepared from the diyne compound (formula 10) being allowed to react with dianion of 3-butyn-1-ol (formula 11) in the presence of copper salt.

The amount of the dianion in 3-butyn-1-ol (formula 11) to be used is favourably 1.0∼2.5 equivalent weight to the amount of diyne compound (formula 10).

The monovalent copper salt is to be used in the necessary amount for catalyst.

The amount of copper (I) chloride to be used is favourably 0.1 ∼ 0.5 equivalent weight to the amount of diyne compound (formula 10).

This step preferably proceeds in a solvent, preferably the ether system solvent especially tetrahydrofuran.

The desirable reaction temperature is 20∼70°C in the case of diyne compound (formula 10) which is allowed to react with anion of 3-butyn-1-ol (formula 11).

### [The step 3]

In summary of this step, the triene compound (formula 13) is prepared from the triyne compound (formula 12) by catalytic reduction with hydrogen gas in the presence of the catalyst and quinoline.

As for the reducing catalyst used in this step, P-2 nickel, Lindlar catalyst, palladium hold on carriers such as calcium sulfate or barium sulfate is a preferable example.

The amount of the catalyst to be used is favourably 0.1∼0.3 equivalent weight to the amount of triyne compound (formula 12).

This reaction proceeds preferably in a solvent selected from the ester system solvent like as ethyl acetate, the alcoholic system solvent like as methanol, the ethanol or the ether system solvent like as tetrahydrofuran, dioxane etc.

As for desirable conditions the hydrogen pressure is 1.0∼1.2 atm and the reaction temperature is 0 ∼40°C.

### [The step 4]

In summary of this step, the mesylate compound (formula 15) is prepared from the triene compound (formula 13) being allowed to react with methanesulfonyl chloride (formula 14) in the presence of the amine.

The amount of methanesulfonyl chloride (formula 14) to be used is favourably 1.0 ∼2.0 equivalent weight to the amount of triene compound (formula 13).

The amine used preferably in the present invention is triethylamine, pyridine and the amount of above amine to be used is favourably 1.5∼3.0 equivalent weight to the amount of triene compound (formula 13).

This reaction preferably proceeds in a solvent selected from the halogen system solvents such as methylene chloride, chloroform or the ether system solvents such as tetrahydrofuran, dioxane etc.

It is desirable that the reaction temperature is-20∼20°C.

### [The step 5]

In summary of this step, the bromide compound (formula 16) is prepared from the mesylate compound (formula 15) which is allowed to react with a bromination agent.

In this step, the bromination agent to be used is favourably lithium bromide, sodium bromide, or the like.

The amount of the bromination agent to be used is favourably 1.5∼5.0 equivalent weight to the amount of mesylate compound (formula 15).

It is preferable that this step is carried out in the presence of a mild basicity salt, for example, sodium hydrogen carbonate in a favourable amount of 0.5∼2.0 equivalent weight to the amount of mesylate compoud (formula 15).

This step preferably proceeds in a solvent selected from the ether system solvents like tetrahydrofuran, dioxane etc.

### [The step 6]

In summary of this step, the tetraene compound (formula 17) is prepared from the bromide compoud (formula 16) which is allowed to react with an alkaline compound.

The alkaline compound to be used is in this step is favourably selected from sodium hydroxide, potassium hydroxide, lithium hydroxide.

The amount of the above alkaline compound to be used is favourably 2.0∼10 equivalent weight to the amount of bromide compound (formula 16).

This step proceeds in a solvent selected from the alcoholic system solvents such as methanol, ethanol, isopropyl alcohol or the mixture of the above alcoholic system solvents in any proportions etc.

It is preferable that the reaction temperature is 30 ∼70°C.

### [The step 7]

In summary of this step, the tetraene alcoholic compound (formula 18) is prepared from the tetraene compound (formula 17) which is allowed to react with an acidic compound in the same amount as the catalyst in the solvent.

The acidic compound to be used favourably in this step is tosylic acid, pyridinium para-toluenesulfonate, acetic acid, hydrochloric acid or the like.

The solvent to be used favourably in this step is selected from the alcoholic system solvents such as methanol, ethanol, the alcholic mixing solvent which was mixed with water or the alcholic system solvent in any proportions etc.

It is preferable that the reaction temperature is 10∼50°C.

### [The step 8]

In summary of this step, the epoxy-alcoholic compound of (5Z,8Z)-cis-2,3-epoxy-5,8,10-undecatrien-1-ol (formula 3) in high optical purity is prepared from tetraene alcoholic compounds (formula 18) carried out by the asymmetric epoxidation by Sharpress et al. (T.Katsuki, K.B.Sharpress, J.Am.Chem.Soc.,102,5974(1980).

The amount of titanium isopropoxide to be used is favourably 0.2∼2.0 equivalent weight to the amount of tetraene alcoholic compoud (formula 18) in this step.

The amount of the type 4A molecular sieves to be used is favourably 0.5 ∼2.0 equivalent weight to the amount of tetraene alcoholic compound (formula 18).

The completion of this reaction can be accelerated by the addition of type 4A molecular sieves.

In this step, D-(-)-diethyl tartarate is used for synthesizing (2R,3S,5Z,8Z)-cis-2,3-epoxy-5,8,10-undecatrien-1-ol (formula 3a)
and L-(+)-diethyl tartarate is used to synthesize (2S,3R,5Z,8Z)-cis-2,3-epoxy-5,8,10-undecatrien-1-ol (formula 3b).

The amount of dietyl tetrate to be used is favourably 1.2∼2.0 equivalent weight to the amount of tetraene alcoholic compound (formula 18).

The amount of tert-buthyl hydroperoxide to be used is favourably 1.2∼2.0 equivalent weight to the amount of tetraene alcoholic compound (formula 18).

It is preferable that the reaction temperature is -10∼30°C.

The optical purity of epoxy alcoholic compound (foumula 3) in this step is 80∼90% e.e, though a slightly different purity may occur depending on the amount of titanium isopropoxide, reaction temperature, the time required for the completion of this reaction etc,.

The optical purity of epoxy alcoholic compound (foumula 3) is determined by high-performance liquid chromatography analysis of the ester derivative with (-)-camphanic acid.

### [The step 9]

In summary of this step, the optically pure 3,5-dinitro-benzoate compound (formula 19) is recrystallized from crude 3,5-dinitro-benzoate derived with 3,5-dinitrobenzoyl chloride and pyridine from the optical active epoxy and the alcoholic compound (formula 3) which is obtained by the above asymmetric epoxidation reaction of Sharpress.

The solvent used for recrystallization of 3,5-dinitrobenzoate compound (formula 19) is favorably exampled in the aliphatic hydrocarbon system solvents like n-hexane, n-pentane for example, the ether system solvents like diethyl ether, tetrahydrofuran or a mixture of any of the above solvents in any proportion etc.

### [The step 10]

To summarize this step, the optically pure epoxy alcoholic compound (formula 3) as described (5Z,8Z)-cis-2,3-epoxy-5,8,10-undecatrien-1-ol (formula 3) is prepared from the optically pure 3,5-dinitrobenzoate compound ( formula 19) decomposed with methanol in the presence of the alkaline compound which is equal to the amount of the catalyst.

Examples of the alkaline compound used preferably in this step are sodium carbonate, sodium hydroxide, potasium hydroxide or the like.

The amount of above alkaline compound to be faborably used is 0.05 ∼0.5 equivalent weight to the amount of 3,5-dinitrobenzoate compound (formula19).

It is preferable that the reaction tempreature is -10∼10°C.

After completion of the reaction, by extraction, concentration and purification using a silica gel chromatography, the optically pure (5Z,8Z)-cis-2,3-epoxy-5,8,10-undecatrien-1-ol (formula 3) is obtained.

The optical purity of (5Z,8Z)-cis-2,3-epoxy-5,8,10-undecatrien-1-ol (formula 3) is determined by high performance liquid chromatography analysis of the ester derovative with (-)-camphanic acid.

Hereinafter, *Hyphantria cunea* attractant compositions according to the present invention are described.

Either (3Z,6Z)-cis-9,10-epoxy-1,3,6-heneicosatriene (formula 1) or (3Z,6Z)-cis-9,10-epoxy-1,3,6-eicosatriene (formula 2) can be used by themselves as captive attractants.

However, if above attractants and known *Hyphantria cunea* sex pheromones are used together at the same time, their captive effect may be increased.

In *Hyphantria cunea* attractant compositions of the present it is not necessary to use all three kinds of known sex pheromones together nor is the use of both (3Z,6Z)-cis-9,10-epoxy-1,3,6-heneicosatriene (formula 1) and (3Z,6Z)-cis-9,10-epoxy-1,3,6-eicosatriene (formula 2) together as the inventive H.cunea sex pheromones necessary. However, it is preferable that (3Z,6Z)-cis-9,10-epoxy-1,3,6-heneicosatriene (formula 1) is included as at least one component of the inventive composition.

The productive examples as embodiments of this invention are described in the following relating to the inventive compositions (3Z,6Z)-cis-9,10-epoxy-1,3,6-heneicosatriene (formula 1), (3Z,6Z)-cis-9,10-epoxy-1,3,6-eicosatriene (formula 2) and (5Z,8Z)-cis-2,3-epoxy-5,8,10-undecatrien-1-ol (formula 3).

Furthermore, *Hyphantria cunea* attractant compositions of the present invention are described with reference to the following tests and comparative tests.

### EXAMPLE

### (Example 1.)

The present Example 1 illustrates the method of producing (3Z,6Z,9S,10R)-cis-9,10-epoxy-1,3,6-heneicosatriene from (2R,3S,5Z,8Z) -cis-2,3-epoxy-5,8,10-undecatrien-1-ol.

(2R,3S,5Z,8Z)-cis-2,3-epoxy-5,8,10-undecatrien-1-ol(0.59g) was solved in pyridine(14ml), and tosylchloride(0.94g) was added to the solution under cooling with ice.

The mixture was stirred for 15 hours at 0°C.

The reaction mixture was poured into the iced water, from which an extrative layer was separated with ether.

The layer of ether was washed by a saturated solution of copper sulfate, a saturated solution of salt in that order, and it was dried by magnesium sulfate; thereafter it was concentrated.

The concentrated substance was purified by silica gel chromatography (35g of silica gel, n-pentane/ether 5:1) to give the reaction product.

The reaction product was 3010, 1595, 1360, 1190, 1175, 970 , 810 (cm ⁻¹) in IR spectrum which was measured by using film method for preparing samples.

As the above result shows, it is confirmed that the reaction product is (2R,3S,5Z,8Z)-cis-2,3-epoxy-5,8,10-undecatrienyltosylate.

The yield amount of reaction product was 0.95g, the yield rate 87%.

The obtained (2R,3S,5Z,8Z)-cis-2,3-epoxy-5,8,10-undecatrienyltosylate (284mg) was solved in ether (7ml), and the solution was dropped into a solution of lithium di(n-decyl) cuprate in ether (0.75M, 3,4ml) at -60°C in a stream of argon.

Then the reaction mixture was stirred for 30 min. at 60 °C, and it was poured into a saturated solution of ammonium chloride. Subsequently, it was stirred for 30 min, the stirred mixture was filtrated, the filtrate was separated from a layer of organic substance and a layer of water, thereafter an extractive layer was separated with ether from the layer of water.

The mixture of the layer of organic substance and the extractive layer of ether from the layer of water was cleaned by water, a saturated solution of salt in that order, and it was dried by magnesium sulfate; thereafter it was concentrated.

The concentrated substance was purified by silica gel chromatography (40g of silica gel, n-pentane/ether 50:1) to give the reaction product.

The reaction product was measured by mass spectrometry.

The result showed that the molecular weight of reaction product was 304.2744, molecular formula was C₂₁H₃₆O(F.W.=304.2766).

The IR spectrum peak for the reaction product were 3090, 3020, 2960, 2930, 2860, 1640, 1590, 1460, 1430, 1380, 1260, 995, 900, 720 (cm ⁻¹).

The measurement was carried out by using film method for preparing samples.

Moreover, the δ amount (ppm) of proton nuclear magnetic resonance (¹H-NMR) spectrum were 0.88 (t;J=6.8Hz, 3H), 1.18 ∼1.55 (m;20H), 2.18 ∼2.29 (m;1H),2.40 (dt;J=6.0, 14.4Hz, 1H), 2.88∼3.00 (m;4H), 5.13 (dd;J=7.0, 17.0Hz,1H), 5.22 (dd;J=2.0, 17.0Hz,1H), 5.41 (dd;J=8.0, 18.0Hz, 1H), 5.45 ∼5.57 (m;2H), 6.03(deformed t;J=11.0Hz, 1H), 6.65(dddd;J=1.1, 10.0, 11.0, 17.0Hz,1H).

The measurement was carried out by dissolving the samples in chloroform-d, and by using 400 MHz of proton nuclear magnetic resonance (¹H-NMR) spectrum .

Furthermore, the melting point of the reaction product was 14 ∼15°C, the refractive index with sodium D light ray at 16°C was 1.4781, the specific rotation with sodium D light ray at 16 °C ;[ α]¹⁶ _{D} was -0.41 ° (C=1.97, in CHCl₃).

From the above results, it is confirmed that the reaction product was (3Z,6Z,9S,10R)- cis-9,10-epoxy-1,3,6-heneicosatriene.

The yield amount of reaction product was 199mg, the yield rate 77 %.

### (Example 2.)

The present Example 2 illustrates the method of producing (3Z,6Z,9Z,10R)-cis-9,10-epoxy-1,3,6-eicosatriene from (2R,3S,5Z,8Z)-cis-2,3-epoxy-5,8,10-undecatrien-1-ol.

(2R,3S,5Z,8Z)-cis-2,3-epoxy-5,8,10-undecatrienyltosylate(184mg) obtained in the same manner as example 1 was dissolved in ether(5*mℓ*), which was dropped into a solution of lithium di(n-nonyl) cuprate in ether (0.72M, 2.3 ml) at-60°C in a stream of argon, thereafter it was stirred for 30min.

The reaction mixture was purified in the same manner as Example 1 to give the reaction product.

The reaction product was measured by mass spectrometry.

The result showed that the molecular weight of reaction product was 290.2581, the molecular formula was C₂₀H₃₄O(F.W.=290.2610).

The IR spectrum peaks of the reaction product were 3090, 3020, 2960 2930, 2860, 1640, 1590, 1460, 1430, 1380, 1260, 995, 900, 720 (cm⁻¹).

The measurement was carried out by using the film method of preparing samples.

Moreover, the δ amounts (ppm) of proton nuclear magnetic resonance (¹H-NMR) spectrum were 0.88 (t;J=6.8Hz, 3H), 1.18∼1.55 (m;18H), 2.18∼2.29 (m;1H),2.40 (dt;J=6.0, 14.4Hz, 1H), 2.88 ∼3.00 (m;4H), 5.13 (dd;J=7.0, 17.0Hz,1H), 5.22 (dd;J=2.0, 17.0Hz, 1H), 5.41 (dd;J=8.0, 18.0Hz, 1H), 5.45 ∼5.57 (m;2H), 6.03 (deformed t; J=11.0Hz, 1H), 6.65 (dddd;J=1.1, 10.0, 11.0, 17.0Hz,1H).

The measurement was carried out by dissolving the samples in chloroform-d, and by using 400 MHz of proton nuclear magnetic resonance (¹H-NMR) spectrum .

Furthermore, the melting point of the reaction product was 2∼3 °C, the refractive index with sodium D light ray at 16°C was 1.4787, the specific rotation with sodium D light ray at 16°C ;[ α]¹⁶ _{D} was -0.57° (C=1.23,in CHCl₃).

The above results confirm that the reaction product was (3Z,6Z,9S,10R)- cis-9,10-epoxy-1,3,6-eicosatriene.

The yield amount of reaction product was 109 mg, the yield rate 68%.

### (Example 3.)

The present Example 3 illustrates the method of producing (3Z,6Z,9R,10S)-cis-9,10-epoxy-1,3,6-eicosatriene from (2S,3R,5Z,8Z) cis-2,3-epoxy-5,8,10-undecatrien-1-ol.

(2S,3R,5Z,8Z)-cis-2,3-epoxy-5,8,10-undecatrienyltosylate(0.78g) was obtained from (2S,3R,5Z,8Z)-cis-2,3-epoxy-5,8,10-undecatrien-1-ol (0.47g) in the same manner as Example 1.

Its IR spectrum was the same as (2R,3S,5Z,8Z)-cis-2,3-epoxy-5,8,10-undecatrienyltosylate which was obtained in Example 1. Furthermore, the obtained (2S,3R,5Z,8Z)-cis-2,3-epoxy-5,8,10-undecatrienyltosylate (240 mg) was treated in the same manner as Example 1 to give the reaction product.

The mass spectrum, the IR spectrum, the proton nuclear magnetic resonance (¹H-NMR) spectrum, the melting point, the refractive index with sodium D light ray at 16 °C of the reaction product was the same as (3Z,6Z,9S,10R)-cis-9,10-epoxy-1,3,6-heneicosatriene which was obtained in Example 1.

But the specific rotation with sodium D light ray at 16 °C; [α]¹⁶ _{D} was +0.43° (C=2.39, in CHCl₃).

As the above results show, it is confirmed that the reaction product was (3Z,6Z,9R,10S)-cis-9,10-epoxy-1,3,6-eicosatriene.

The yield amount of reaction product was 164 mg.

### (Example 4.)

The present Example 4 illustrates the method for producing (3Z,6Z,9R,10S)-cis-9,10-epoxy-1,3,6-eicosatriene from (2S,3R,5Z,8Z)-cis-2,3-epoxy-5,8,10-undecatrien-1-ol.

The (2S,3R,5Z,8Z)-cis-2,3-epoxy-5,8,10-undecatrienyltosylate (147mg) was treated in the same manner as Example 2 to give the reaction product.

The mass spectrum, the IR spectrum, the proton nuclear magnetic resonance (¹H-NMR) spectrum, the melting point, the refractive index with sodium D light ray at 16 °C of the reaction product was the same as (3Z,6Z,9S,10R)-cis -9,10-epoxy-1,3,6-eicosatriene which was obtained in Example 2.

But the specific rotation with sodium D light ray at 16 °C ; [α]¹⁶ _{D} was+0.62° (C=2.34, in CHCl₃).

As the above results confirm, the reaction product was (3Z,6Z,9R,10S)-cis-9,10-epoxy-1,3,6-eicosatriene.

The yield amount of the reaction product was 95 mg, the yield rate 74 %.

### (Example 5.)

The present Example 5 illustrates the method of producing (2R,3S,5Z,8Z)-cis-2,3-epoxy-5,8,10-undecatrien-1-ol.

### (The step 1.)

3-(tetrahydropyranyloxy)-1-propyne (58.9g) was dissolved in tetrahydrofuran in a stream of argon, which was dropped into a solution of ethylmagnesium bromide in tetrahydrofuran (2.0M, 210 ml) at room temperature.

The mixture was stirred for 1 hour at 50°C, after which copper (I) chloride (4.1g) was added ,and the mixture stirred for 20min.at 40°C. Subsequently, a solution of 1.4-dichloro-2-butyne (49.2g) in tetrahydrofuran (100*m*ℓ) was poured into the mixture at 5°C.

The reaction mixture was heated to 40 °C, and stirred for 12 hours.

Then, it was cooled to room temperature and poured into an ammonium chloride solvent (400ml), from which an extractive layer was separated with ether .

The layer of ether was washed by a saturated solution of salt it has dried by magnesium sulfate. Thereafter it was concentrated.

The concentrated substance was purified by using silica gel chromatography (800g of silica gel, n-pentane/ether 15:1) to give the reaction product.

The IR spectrum peaks of the reaction product were 2300, 2230, 1120, 1025 (cm ⁻¹).

The measurement was carried out by using the film method for preparing samples.

Moreover, the δ amounts (ppm) of proton nuclear magnetic resonance (¹H-NMR) spectrum were 1.20∼2.20 (m;6H), 3.29 (t;J=2, 9Hz, 2H), 3.35 ∼4.00(m;2H), 4.12 (t;J=2.9Hz, 2H), 4.28 (dd;J=4.7, 2.9Hz,2H), 4.80 (m;1H).

The measurement was carried out by dissolving the samples in chloroform-d, and by using 100 MHz of proton nuclear magnetic resonance (¹H-NMR) spectrum . Furthermore, the refractive index with sodium D light ray at 19 °C was 1.5096.

As the above results demonstrate, it is confirmed that the reaction product was 1-chloro-7-(2-tetrahydropyranyloxy)-2,5-heptadiyne.

The yield amount of reaction product was 52.6 g, the yield rate 58%.

### (The step 2.)

3-butyn-1-ol(24.4g)was dissolved in tetrahydrofuran(100ml) in a stream of argon, and the solution was dropped into a solution of ethylmagnesium bromide in tetrahydrofuran (2.0M,382.5 ml) at 20 °C.

The mixture was stirred for 1 hour at 60°C, after which copper (I) chloride (7.6g) was added, and stirred for 1 hour at 35 °C.

1-chloro-7-(2-tetrahydropyranyloxy)-2,5-heptadiyne (52.6g) was dissolved in tetrahydrofuran(150ml) which was added to the stirred solution at 35°C.

Then it was stirred for 15 hours at 40 °C, and for 5 hours at 60 °C.

The reaction mixture was poured into a saturated solution of ammonium chloride solvent, from which an extractive layer was sepatated with ether.

The layer of ether was washed by a saturated solution of salt, and it was dried by magnesium sulfate. Thereafter it was concentrated.

The concentrated substance was purified by using silica gel chromatography (350g of silica gel, n-pentane/ether1:1) to obtain the reaction product.

The IR spectrum peaks of the reaction product were 3450, 2250, 1120, 1060, 1040, 1025 (cm ⁻¹).

The measurement of it was carried out by using the film method for preparing samples.

Moreover, the δ amounts (ppm) of proton nuclear magnetic resonance (¹H-NMR) spectrum were 1.30∼2.10 (m;6H), 1.91(br;OH), 2.30∼2.57 (m;2H), 3.00 ∼3.29(m;4H),3.35 ∼4.02(m;2H), 3.72 (t;J=6.8Hz,2H), 4.24 (dt;J=2.3, 16.0Hz,2H), 4.80 (m;1H).

The measurement was carried out by dissolving the samples in chloroform-d, and by using 100 MHz of proton nuclear magnetic resonance (¹H-NMR) spectrum .

As the above results show, it is confirmed that the reaction product was 11-(2-tetrahydropyranyloxy)-3,6,9-undecatriyn-1-ol.

The yield amount of reaction product was 49.6 g.

### (The step 3.)

11-(2-tetrahydropyranyloxy)-3,6,9-undecatriyn-1-ol(49.6g) was dissolved in ethylacetate(500ml), to which Lindlar catalyst(8.5g)and quinoline (8ml) were added, then the mixture was stirred furiously in a stream of hydrogen.

On completion of the consumption of hydrogen the reduction solution was filtrated, and the filtrate was concentrated.

The concentrate was dissolved in ether, which was cleaned by diluted hydrochloric acid, water, a saturated solution of salt in order, then the layer of ether was washed by a saturated solution of salt and dried by magnesium sulfate. Thereafter it was concentrated.

The concentrated substance was purified by using silica gel chromatography (900g of silica gel, n-pentane/ether2:1) to give the reaction product.

The IR spectrum peaks of the reaction product were 3430, 3025, 1120, 1080, 1055, 1025 (cm ⁻¹).

The measurement was carried out by using the film method for preparing samples.

Moreover, the δ amounts (ppm) of proton nuclear magnetic resonance (¹H-NMR) spectrum were 1.10~2.10 (m;6H), 1.77(br;OH), 2.35 (dd;J=5.8, 8.7 Hz,2H), 2.50 ∼3.10(m;4H),3.65 (t;J=5.8Hz,2H) 3.20∼4.50(m;4H), 4.65(m;1H), 5.15∼5.80 (m;6H).

The measurement was carried out by dissolving the samples in chloroform-d, and by using 100 MHz of proton nuclear magnetic resonance (¹H-NMR) spectrum .

Furthermore, the refractive index with sodium D light ray at 18 °C was 1.4953.

As the above results show, it is confirmed that the reaction product was (3Z,6Z,9Z)-11-(2-tetrahydropyranyloxy)-3,6,9-undecatrien-1-ol.

The yield amount of reaction product was 43.9 g, the yield rate 86%.

### (The step 4.and The step 5.)

(3Z,6Z,9Z)-11-(2-tetraydrophyranyloxy)-3,6,9-undecatrien-1-ol (43.9g) was dissolved in methylene chloride(460ml), to which triethyl amine(330ml) was added, and then the mixture was cooled with ice, Thereafter, methanesulfonyl chloride(22.7g) was added dropwise thereto.

It was stirred for 10min., then the reaction mixture was cleaned by water, cold diluted hydrochloric acid, a saturated solution of sodium bicarbonate and water, respectively. Afterwards, it was dried by magnesium sulfate; thereafter it was concentrated to give a mesylate compound(57.0g).

The obtained crude mesylate compound was dissolved in a solution of tetrahydrofuran (630ml) to which lithum chloride anhydride (71.7g) and sodium bicarbonate(35.3g) were added, then it was stirred for 18 hours.

Thereafter the reaction mixture was concentrated , and it was dissolved in ether.

The solution of ether was filtrated, and the filtrate was washed by a saturated solution of salt, before being dried by magnesium sulfate and then being concentrated.

The concentrated substance was purified by using silica gel chromatography (600g of silica gel, n-pentane/ether20:1) to give the reaction product.

The IR spectrum peaks of the reaction product were 3025, 1120, 1020 (cm ⁻¹).

The measurement of it was carried out by using the film method for preparing samples.

Moreover, the δ amounts(ppm) of proton nuclear magnetic resonance (¹H-NMR) spectrum were 1.20∼2.20 (m;6H), 2.40∼3.10 (m;6H), 3.38 (ddd;J=0.6,7.0,7.0 Hz,2H), 3.45 ∼4.07(m;2H), 4.07∼ 4.30(m;2H), 4.63(m;1H), 5.17∼5.80 (m;6H).

The measurement was carried out by dissolving the samples in chloroform-d, and by using 90 MHz of proton nuclear magnetic resonance (¹H-NMR) spectrum .

Further, the refractive index with sodium D light ray at 20°C was 1.5078.

As the above results confirm the reduction product was (3Z,6Z,9Z)-1-bromo-11-(2-tetrahydropyranyloxy)-3,6,9-undecatriene.

The yield amount of reaction product was 47.3g, the yield rate 87%.

### ( The step 6.)

Potassium hydroxide(40.5g) was dissolved in a mixture of methanol (1500ml) and ethanol (3000ml), to which (3Z,6Z,9Z)-1-bromo-11-(2-tetrahydropyranyloxy)-3,6,9-undecatriene(47.3g) was added, and the mixture was stirred 20min at 65 °C.

The reaction mixture was concentrated, ether and water were poured therein, and it was stirred for extraction.

The layer of ether was washed by a saturated solution of salt, and it was dried by magnesium sulfate; thereafter it was concentrated.

The concentrated substance was purified by using silica gel chromatography (1100g of silica gel, n-pentane/ether50:1) to give the reaction product.

The IR spectrum peaks of the reaction product were 3090, 3025, 1640,1590,1120,1025,905 (cm ⁻¹).

The measurement was carried out by using the film method for preparing samples.

Moreover, the δ amounts (ppm) of proton nuclear magnetic resonance (¹H-NMR) spectrum were 1.10∼2.30 (m;6H), 2.60∼3.10 (m;4H), 3.30∼4.45 (m;4H), 4.65 (m;1H), 4.90 ∼5.80(m;7H), 6.03 (dd;J=10.9, 10.9 Hz,1H), 6.68 (dt;J=10.9,16.8 Hz,1H) .

The measurement was carried out by dissolving the samples in chloroform-d, and by using 100 MHz of proton nuclear magnetic resonance (¹H-NMR) spectrum .

Furthermore, the refractive index with sodium D light ray at 20 °C was 1.5021.

As the above result confirm, the reaction product was (2Z,5Z,8Z)-1-(2-tetrahydropyranyloxy)-2,5,8,10-undecatetraene.

The yield amount of reaction product was 21.1g, the yield rate 59 %.

### ( The step 7. )

(2Z,5Z,8Z)-1-(2-tetrahydropyranyloxy)-2,5,8,10-undecatetraene (21.1g) was dissolved in methanol (150 ml), to which pyridinium paratoluene sulfonate(4g) was added and it was stirred for 24 hours at room temperature.

Then the reaction mixture was concentrated, and concentrated substance was dissolved in ether.

The solution of ether was washed by a saturated solution of sodium bicarbonate and a saturated solution of salt, in that order. It was dried by magnesium sulfate. Thereafter it was concentrated.

The concentrated substance was purified by using silica gel chromatography (700g of silica gel, n-pentane/ether4:1) to give the reaction product.

The IR spectrum peaks of the reaction product were 3350, 3040, 1640,1590,1000, 905 (cm ⁻¹).

The measurement was carried out by using the film method for preparing samples.

Moreover, the δ amounts(ppm) of proton nuclear magnetic resonance (¹H-NMR) spectrum were 2.60 ∼3.10 (m;4H),4.25 (d;J=5.5 Hz,2H), 4.90∼5.85 (m;7H),6.03 (dd;J=10.9, 10.9 Hz,1H),6.68 (dddd;J=0.9,9.8, 10.9,16.8 Hz,1H).

The measurement was carried out by dissolving the samples in chloroform-d, and by using 100 MHz of proton nuclear magnetic resonance (¹H-NMR) spectrum . Furthermore, the refractive index with sodium D light ray at 20°C was 1.5167.

As the above results confirm, the reaction product was (2Z,5Z,8Z)-2,5,8,10-undecatetraen-1-ol.

The yield amount of reaction product was 13.0g, the yield rate 93 %.

### ( The step 8.)

In a stream of argon, titanium isopropoxide(10.6g),D-(-)-diethyltartarate (11.5g) were in order added to the mixture of methylene chloride(330ml) and the type of molecular sieves 4A (9.9g) at -23 °C, and it was stirred for 30min.

Further, the solution of (2Z,5Z,8Z)-2,5,8,10-undecatetraen-1-ol (6.2g) in methylen chloride(30ml), and the solution of *tert*-butylhydroperoxide in methylene chloride(5.7M, 13.1 ml) were added to above the solution at -25 °C.

Then the reaction mixture was stirred for 15 hours at -25 °C.

10% solution of tartaric acid(190ml) was added thereto and it was held at temperature and stirred for 1 hour.

The solution of reaction mixture was separated, the layer of methylene chloride was diluted with n-hexane , then it was washed by water several times, and dried by magnesium sulfate. Thereafter it was concentrated.

The concentrated substance was purified by using silica gel chromatography (700g of silica gel, n-pentane/ether5:1∼3:1) to give the reaction product.

The reaction product was measured by mass spectrometry.

The result showed that the molecular weight of reaction product was 180.22. The molecular formula was C₁₁H₁₆O₂(F.W.=180.1150).

The IR spectrum peaks of the reaction product were 3430, 3090, 3020, 1640, 1590, 1040, 905 (cm ⁻¹).

The measurement was carried out by using the film method for preparing samples.

Moreover, the δ amounts (ppm) of proton nuclear magnetic resonance (¹H-NMR) spectrum were 1.20∼1.90 (m;2H),1.90∼2.70 (m;2H), 2.70 ∼3.30 (m;2H),3.50∼4.10 (m;2H),4.90∼5.85 (m;5H),6.04 (dd;J=10.9,10.9 Hz,2H), 6.65 (dddd;J=0.9, 9.8,10.9,16.8 Hz,1H).

The measurement was carried out by dissolving the samples in chloroform-d, and by using 100 MHz of proton nuclear magnetic resonance (¹H-NMR) spectrum .

Furthermore, the refractive index with sodium D light ray at 19 °C was 1.5112, the specific rotation with sodium D light ray at 19°C; [ α]¹⁹ _{D} was + 5.53 ° (C=0.85, in CHCl₃), and optical purity was 87.8%e.e.

As the above results show, it is confirmed that the reaction product was (2R,3S,5Z,8Z)-cis-2,3-epoxy-5,8,10-undecatrien-1-ol.

The yield amount of reaction product was 4.1g, the yield rate 60%.

### ( The step 9.)

A solution of (2R,3S,5Z,8Z)-cis-2,3-epoxy-5,8,10-undecatrien-1-ol(3.85g,87.8%e.e.) in ether (85 ml) to which pyridine (24ml) and 3,5-dinitrobenzoylchloride(6.23g) were added under cooling with ice, and it was stirred for 12 hours at 0 °C.

The reaction mixture was poured into iced water, from which an extractive layer was separated with ether.

The layer of ether was washed by a saturated solution of coppersulfate and a saturated solution of salt in that order, and it was dried by magnesium sulfate. Thereafter it was concentrated.

The concentrated substance was purified by recrystallizing with a solvent of n-pentane/ether(3:1 ∼1:1 ) several times to give yellowish imbricate crystallization.

The IR spectrum peaks of the reaction product were 3100, 3020, 1725, 1630, 1595, 1545, 1345, 1280, 1180, 910, 730, 720 (cm ⁻¹).

The measurement was carried out by using film the method for preparing samples.

Moreover, the δ amounts(ppm) of proton nuclear magnetic resonance (¹H-NMR) spectrum were 2.10 ∼2.65 (m;2H),2.65∼3.60 (m;4H), 4.50 (d;J=6.0Hz,1H),4.61(d;J=4.6Hz,1H),4.90 ∼5.75 (m;5H),6.00 (t;J=10.9Hz,1H), 6.60 (dd;J=10.9,16.8Hz,1H) .

The measurement was carried out by dissolving the samples in chloroform-d, and by using 60 MHz of proton nuclear magnetic resonance (¹H-NMR) spectrum . Furthermore, the melting point of the reaction product was 33∼ 33.5°C, the specific rotation with sodium D light ray at 16 °C [ α]¹⁶ _{D} was +19.3° (C=0.58, in CHCl₃).

As the above results show, it is confirmed that the reaction product was (2R,3S,5Z,8Z)-1-(3',5'-dinitrobenzoyloxy)-cis-2,3-epoxy-5,8,10-undecatriene.

The yield amount of reaction product was 1.61g, the yield rate 20 %.

### ( The step 10.)

A solution of (2R,3S,5Z,8Z)-1-(3',5'-dinitrobenzoyloxy)-cis-2,3-epoxy-5,8,10-undecatriene (1.49g) in methanol(15ml) to which potassium carbonate (0.15g) was added under cooling with ice was stirred for 1 hour at 0 °C.

The reaction mixture was concentrated, then it was diluted with water, from which an extractive layer was separated with ether.

The layer of ether was washed by water,a saturated solution of salt in order,and it was dried by magnesium sulfate. Thereafter it was concentrated.

The concentrated substance was purified by using silica gel chromatography (45g of silica gel, n-pentane/ether 4:1∼2:1) to give the reaction product.

The mass spectrum, the IR spectrum, the proton nuclear magnetic resonance (¹H-NMR) spectrum and the refractive index with sodium D light ray at 19 °C of the reaction product were the same as (2R,3S,5Z,8Z)-cis-2,3-epoxy-5,8,10-undecatrien-1-ol which was obtained at The step 8.

But the specific rotation with sodium D light ray at 18 °C; [ α]¹⁸ _{D} was+6.20° (C=1.66, in CHCl₃), and optical purity was 100%e.e.

As the above results show, it is confirmed that the reaction product was (2R,3S,5Z,8Z)-cis-2,3-epoxy-5,8,10-undecatrien-1-ol .

The yield amount of reaction product was 670mg, the yield rate 93 %.

### (Example 6.)

The present Example 6 illustrates the method for producing (2S,3R,5Z,8Z)-cis-2,3-epoxy-5,8,10-urdecatrien-1-ol.

(2Z,5Z,8Z)-2,5,8,10-undecatetraen-1-ol(6.1g) was treated in the same manner as The step 8(Example 5) except for using L-(+)-diethyltartrate which was substituted for D-(-)-diethyltartrate to give the reaction product.

The IR spectrum, the proton nuclear magnetic resonance (¹H-NMR) spectrum and the refractive index with sodium D light ray at 19°C of the reaction product were the same as (2R,3S,5Z,8Z)-cis-2,3-epoxy-2,5,8,10-undecatrien-1-ol which was obtained at The step 8.

But the specific rotation with sodium D light ray at 20 °C [ α]²⁰ _{D} was -5.37° (C=1.09, in CHCl₃), and optical purity was 89.6%e.e.

As the above results show, it is confirmed that the reaction product was (2S,3R,5Z,8Z)-cis-2,3-epoxy-5,8,10-undecatrien-1-ol .

The yield amount of reaction product was 4.0g, the yield rate 59%.

Furthermore, (2S,3R,5Z,8Z)-cis-2,3-epoxy-5,8,10-undecatriene-1-ol (3.72g) was treated in the same manner as The step 9(exsample 5) to give the reaction product.

The IR spectrum, the proton nuclear magnetic resonance (¹H-NMR) spectrum and the melting point of the reaction product were the same as (2R,3S,5Z,8Z)-1-(3',5'-dinitrobenzoyloxy)-cis-2,3-epoxy-5,8,10-undecatriene which was obtained at The step 9.

But the specific rotation with sodium D light ray at 13 °C [ α]¹³ _{D} was -19.1° (C=1.14, in CHCl₃).

As the above results show, it is confirmed that the reaction product was (2R,3S,5Z,8Z)-1-(3',5'-dinitrobenzoyloxy)-cis-2,3-epoxy-5,8,10-undecatriene.

The yield amount of reaction product was 1.39g, the yield rate 18 %.

Moreover (2R,3S,5Z,8Z)-1-(3',5'-dinitrobenzoyloxy)-cis-2,3-epoxy -5,8,10-undecatriene(1.24g) was treated in the came manner as The step 10 (Example 5) to give the reaction product.

The IR spectrum, the proton nuclear magnetic resonance (¹H-NMR) spectrum and the refractive index with sodium D light ray at 18 °C of the reaction product were the same as (2R,3S,5Z,8Z)-cis-2,3-epoxy-5,8,10 -undecatrien-1-ol which was obtained at The step 10.

But the specific rotation with sodium D light ray at 16 °C ; [ α]¹⁶ _{D} was -6.28° (C=1.95, in CHCl₃), and optical purity was 100%e.e.

As the above results show, it is confirmed that the reaction product was (2S,3R,5Z,8Z)-cis-2,3-epoxy-5,8,10-undecatrien-1-ol .

The yield amount of reaction product was 0.55g, the yield rate 92 %.

### (Tests and Comparative tests)

The number of caught *Hyphantria cunea* was researched by using the attractant compositions for *Hyphantria cunea* containing sex pheromones of the invention which were derived from the above Examples.

The sex pheromones combined as described in following Table 1 were involved in trianuglar filter paper ( the sides of triangle were severally about 2cm length) , these were adhered to adhesive plates.

The trap test was conducted at Osaka City ( I : 21st July 1988, 3:00∼5:00) and Ibaraki City ( II : 28th August 1988,4:45 ∼5:30, III : 29th August 1988,4:45 ∼5:30, IV: 30th August 1988,4:45 ∼5:30), the manner of the trap test was that the above adhesive plates were placed in different wire nets which were put on a street tree or a street.

Results of these tests are described in following Table 1.

| | Combination of various pheromones | | | | | Number of caught *Hyphantria cunea* | | | |
|---|---|---|---|---|---|---|---|---|---|
| | 1 | 2 | 3 | 4 | 5 | I | II | III | IV |
| Test Example | ○ | ○ | ○ | ○ | ○ | 8 | 25 | 14 | 18 |
| | ○ | ○ | ○ | ○ | - | 8 | 20 | 10 | 12 |
| | ○ | ○ | - | ○ | - | 6 | 18 | 7 | 12 |
| Comparison | ○ | ○ | ○ | - | - | 1 | 4 | 2 | 1 |
| | ○ | ○ | - | - | - | 0 | 2 | 1 | 0 |
| Note; 1: (9Z,12Z)-9,12-octadecadienal(0.05 mg) 2: (9Z,12Z,15Z)-9,12,15-octadecatrienal(0.4mg) 3: (3Z,6Z)-9,10-epoxy-3.6-heneicosadiene(1.05mg) 4: (3Z,6Z)-9,10-epoxy-1,3,6-heneicosatrierie(0.42 mg) 5: (3Z,6Z)-9,10-epoxy-1,3,6-eicosatriene (0.015mg) | | | | | | | | | |

The above results show that the attractant compositions for *Hyphantris cunea* containing (3Z,6Z)-9,10-epoxy-1,3,6-heneicosatriene, or both (3Z,6Z)-9,10-epoxy-1,3,6-heneicosatriene and (3Z,6Z)-9,10-epoxy-1,3,6-eicosatriene of the invention caused a five-fold increase in the number of H. cunea which were caught as compared with the attractant compositions for *Hyphantria cunea* containing the known sex pheromons.

### ( Effect of the Invention)

As described hereinbefore, the present invention shows the following effects.

The attractant compositions for *Hyphantria cunea* containing said pheromones of this invention are compositions with high attraction effect.

## Claims

1. The attractant compositions for *Hyphantria cunea* including (3Z,6Z)-cis-9,10-epoxy-1,3,6-heneicosatriene (formula 1), (9Z,12Z)-9,12-octadecadien-1-al (formula 4) and (9Z,12Z,15Z)-9,12,15-octadeca -trien-1-al(formula 5).

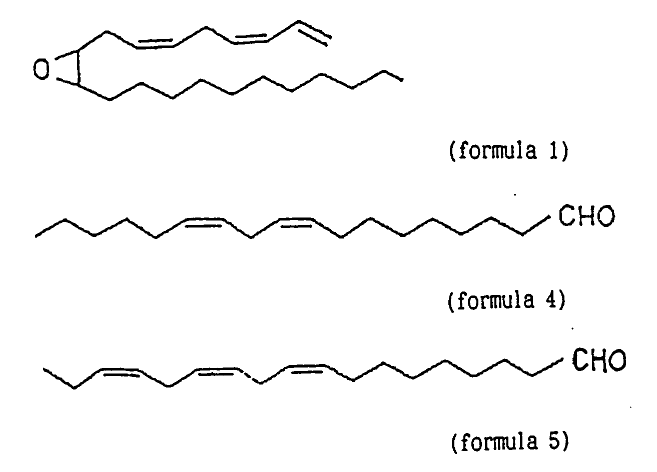

2. The attractant compositions for *Hyphantria cunea* as set forth in claim 1, which is characterized in that (3Z,6Z)-cis-9,10-epoxy-3,6-heneicosadiene (formula 6) is included 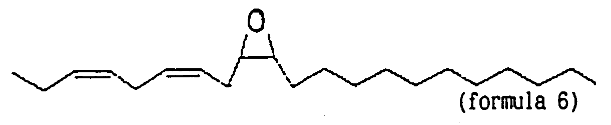 wherein
(3Z,6Z)-3,6-9,10-epoxyeicosadiene,
(3Z,6Z,9Z)-3,6,9-heneicosatriene, and
(3Z,6Z,9Z)-1,3,6,9-heneicosatetraene. are excluded.

3. The attractant compositions for *Hyphantria cunea* as set forth in claim 1 or 2 which is characterized in that (3Z,6Z)-cis-9,10 -epoxy-1,3,6-eicosatriene (formula 2) is included

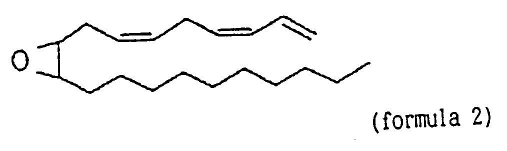

4. The attractant compositions for *Hyphantria cunea* as set forth in any one of the claims 1 to 3 which is characterized in that (3Z,6Z)-cis-9,10-epoxy-1,3,6-heneicosatriene (formula 1) as the (9S,10R) enantiomer of heneicosatriene is included.

5. The attractant compositions for *Hyphantria cunea* as set forth in claim 3 or 4 which is characterized in that (3Z,6Z)-cis-9,10-epoxy-1,3,6-eicosatriene (formula 2) as the (9S,10R) enantiomer of eicosatriene is included.

6. The use of any of the compositions according to claims 1 to 5 for the protection of trees, farm and food products from Hyphantria cunea.

## Patentansprüche

1. Lockstoff-Zusammensetzungen für Hyphantria cunea, die (3Z,6Z)-cis-9,10-Epoxy-1,3,6-heneicosatrien (Formel 1), (9Z,12Z)-9,12-Octadecadien-1-al (Formel 4) und (9Z,12Z,15Z)-9,12,15-Octadecatrien-1-al (Formel 5) enthalten.

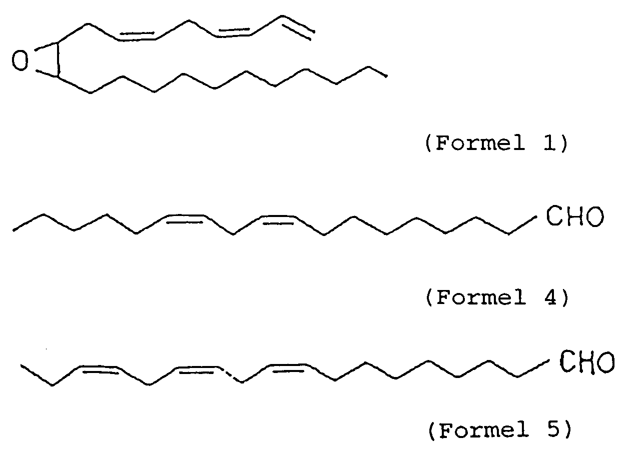

2. Lockstoff-Zusammensetzungen für Hyphantria cunea gemäß Anspruch 1,
dadurch **gekennzeichnet**, daß sie (3Z,6Z)-cis-9,10-Epoxy-3,6-heneicosadien (Formel 6) enthalten, 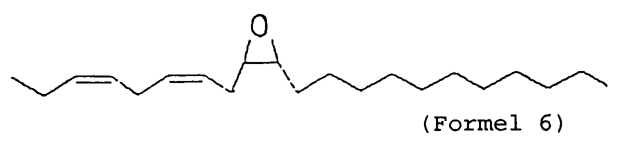 worin
(3Z,6Z)-3,6-9,10-Epoxyeicosadien, (3Z,6Z,9Z)-3,6,9-Heneicosatrien, und (3Z,6Z,9Z)-1,3,6,9-Heneicosatetraen ausgenommen sind.

3. Lockstoff-Zusammensetzungen für Hyphantria cunea gemäß der Ansprüche 1 oder 2,
dadurch **gekennzeichnet**, daß sie (3Z,6Z)-cis-9,10-Epoxy-1,3,6-eicosatrien (Formel 2) enthalten.

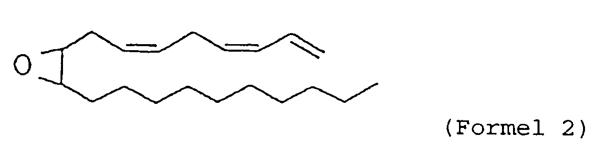

4. Lockstoff-Zusammensetzungen für Hyphantria cunea gemäß einem der Ansprüche 1 bis 3,
dadurch **gekennzeichnet**, daß sie (3Z,6Z)-cis-9,10-Epoxy-1,3,6-heneicosatrien (Formel 1) als (9S,10R)-Enantiomer enthalten.

5. Lockstoff-Zusammensetzungen für Hyphantria cunea gemäß der Ansprüche 3 oder 4,
dadurch **gekennzeichnet**, daß sie (3Z,6Z)-cis-9,10-Epoxy-1,3,6-eicosatrien (Formel 2) als (9S,10R)-Enantiomer enthalten.

6. Verwendung einer der Zusammensetzungen gemäß einem der Ansprüche 1 bis 5 zum Schutz von Bäumen, der Landwirtschaft und von Nahrungsmittelprodukten vor Hyphantria cunea.

## Revendications

1. Compositions attractrices pour *Hyphantria cunea* comprenant du (3Z,6Z)-cis-9,10-époxy-1,3,6-hénéicosatriène (formule 1), du (9Z,12Z)-9,12-octadécadièn-1-al (formule 4) et du (9Z,12Z,15Z)-9,12,15-octadécatrièn-1-al (formule 5).

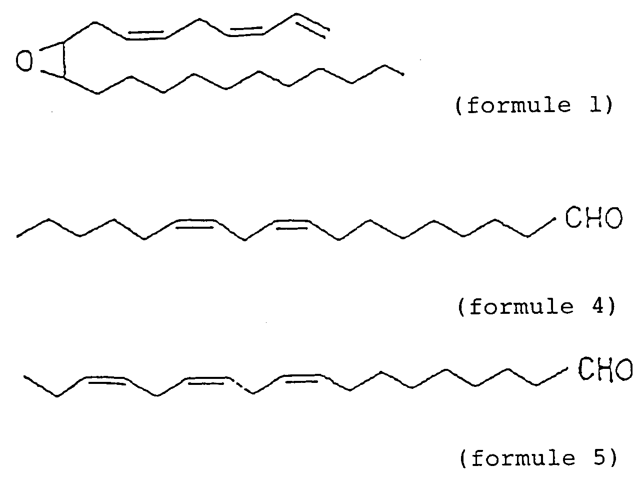

2. Compositions attractrices pour *Hyphantria cunea* selon la revendication 1, caractérisées en ce qu'elles contiennent du (3Z,6Z)-cis-9,10-époxy-3,6-hénéicosadiène (formule 6) 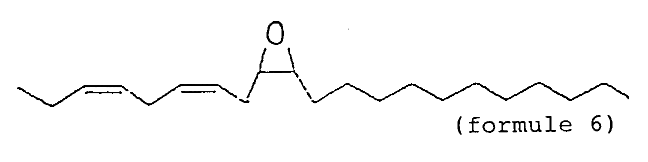 dont
le (3Z,6Z)-3,6-9,10-époxyéicosadiène,
le (3Z,6Z,9Z)-3,6,9-hénéicosatriène,et
le (3Z,6Z,9Z)-1,3,6,9-hénéicosatétraène sont exclus.

3. Compositions attractrices pour *Hyphantria cunea* selon la revendication 1 ou 2, caractérisées en ce qu'elle contiennent du (3Z,6Z)-cis-9,10-époxy-1,3,6-éicosatriène (formule 2).

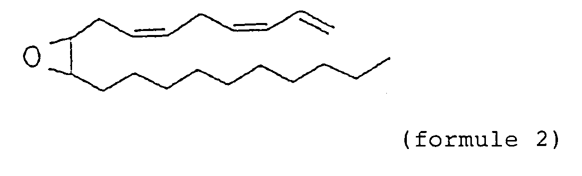

4. Compositions attractrices pour *Hyphantria cunea* selon l'une quelconque des revendications 1 à 3, caractérisées en ce qu'elles contiennent du (3Z,6Z)-cis-9,10-époxy-1,3,6-hènéicosatriène (formule 1) en tant qu'énantiomère (9S,10R) de l'hénéicosatriène.

5. Compositions attractrices pour *Hyphantria cunea* selon la revendication 3 ou 4, caractérisées en ce qu'elles contiennent du (3Z,6Z)-cis-9,10-époxy-1,3,6-éicosatriène (formule 2) en tant qu'énantiomère (9S,10R) de l'éicosatriène.

6. Utilisation d'une quelconque des compositions selon les revendications 1 à 5 pour la protection d'arbres, de produits de ferme et alimentaires envers Hyphantria cunea.
